# EUROPEAN PATENT APPLICATION

(11) **EP 3 643 351 A1**
(43) Date of publication of application: **29.04.2020**
(21) Application number: 18820775.7
(22) Date of filing: 20.06.2018
(51) Int. Cl.: A61M 25/10

(54) **BALLOON CATHETER**

(30) Priority: 23.06.2017 JP 2017122767
(71) Applicant: Goodman Co., Ltd., Aichi 460-0008 (JP)
(72) Inventor: OGAWA, Tomokazu, Seto-shi Aichi 489-0976 (JP); IWANO, Kenshi, Seto-shi Aichi 489-0976 (JP)
(74) Representative: Epping - Hermann - Fischer
(86) International application number: PCT/JP2018/023413
(87) International publication number: WO 2018/235852

(57) **Abstract**

A balloon catheter characterized by being provided with: a catheter shaft (2) extending from a proximal end to a distal end; a balloon (3) which is connected to the catheter shaft (2) and includes an inflation region (33) that can be inflated; a linear member (4) comprising a distal end portion positioned on the distal end side of the inflation region (33), and a proximal end portion (42) positioned on the proximal end side of the inflation region (33), at least one of the distal end portion and the proximal end portion (42) being movable in a direction of extension of the catheter shaft (2); and a first urging member (5) with which, of the distal end portion and the proximal end portion (42), a movement portion that is movable in the direction of extension can be urged in a direction away from the inflation region (33) of the balloon (3), and which is partly bonded to the catheter shaft (2).

## Description

### Technical Field

The present invention relates to a balloon catheter.

### Background Art

A balloon catheter is known that is used in treatments that dilate a constricted location of a blood vessel. Patent Literature 1 discloses a balloon catheter that is provided with a catheter tube, a balloon, a fixed cone-shaped portion, and three linear members. The catheter tube (sometimes also referred to as a "catheter shaft") has an inner tube and an outer tube. The balloon is joined to the outer tube and the inner tube. The balloon inflates when a compressed fluid is supplied. The fixed cone-shaped portion is elastically deformable, and a distal end side thereof is joined to a distal end of the inner tube. The three linear members are disposed on the outer peripheral side of the balloon. The distal end side of the three linear members is joined to a proximal end side of the fixed cone-shaped portion. The proximal end side of each of the three linear members is movable along an extending direction of the catheter tube. An urging spring is provided between the proximal end side of the three linear members and a stopper provided on the outer tube.

The three linear members move in a direction away from the inner tube in accordance with the inflation of the balloon. In this case, the proximal end side of each of the three linear members moves to the balloon side and compresses the urging spring. Meanwhile, in accordance with the deflation of the balloon, the three linear members move in a direction approaching the inner tube. In this case, the proximal end side of each of the three linear members receives an urging force of the urging spring and moves to the opposite side from the balloon.

### Citation List

### Patent Literature

Patent Literature 1: WO2012/029109 A1

### Summary of Invention

In the balloon catheter described in Patent Literature 1, the urging spring is not joined to the outer tube and is slidably provided. Sometimes, a member, such as a stent or the like, is placed inside the blood vessel. When the urging spring is not joined to the outer tube, there is a possibility that the urging spring may become caught on the stent or the like inside the blood vessel and may come apart from the outer tube.

It is an object of the present invention to provide a balloon catheter that is provided with an urging member that does not easily come off.

A balloon catheter according to an aspect of the present invention is characterized by including: a catheter shaft extending from a proximal end to a distal end; a balloon connected to the catheter shaft and including an inflation region that can be inflated; a linear member including a distal end portion positioned further toward the distal end side than the inflation region, and a proximal end portion positioned further toward the proximal end side than the inflation region, at least one of the distal end portion and the proximal end portion being movable along an extending direction of the catheter shaft; and an urging member configured to be capable of urging a movement portion, which is one of the distal end portion and the proximal end portion and which is movable in the extending direction, in a direction away from the inflation region of the balloon, the urging member being partially bonded to the catheter shaft.

In the balloon catheter, when the balloon inflates, the linear member moves to the outside. Therefore, when the balloon inflates in a state in which the balloon is disposed at a constricted location of a blood vessel, the balloon catheter can cause the linear member to act on the constricted location of the blood vessel. Of the distal end portion and the proximal end portion of the linear member, the movement portion that is movable in the extending direction moves to the balloon side in accordance with the inflation of the balloon. Thus, the balloon catheter can suppress the inflation of the balloon from being obstructed by the linear member.

The urging member urges the movement portion in the direction away from the inflation region of the balloon. Therefore, when the balloon returns from the inflated state to the deflated state, the movement portion of the linear member moves in the direction away from the inflation region of the balloon, in response to the urging force received from the urging member. In this case, the warping of the linear member is suppressed when the balloon is in the deflated state. Therefore, the balloon catheter can suppress crossability (passing ability) from deteriorating due to the warping of the linear member.

Further, a part of the urging member is bonded to the catheter shaft. Therefore, even when the urging member becomes caught on a stent or the like placed inside the blood vessel, it is possible to inhibit the urging member from coming apart from the catheter shaft.

In the aspect, the movement portion may be the proximal end portion. In this case, the balloon catheter can suppress the outer diameter on the distal end side from being increased by the urging member. Therefore, the balloon catheter can improve the crossability when the balloon is caused to move toward the constricted location of the blood vessel.

In the aspect, the linear member may be bonded to the catheter shaft or the balloon at the distal end portion. In this case, for example, in comparison to a case in which an elastically deformable member is provided between the distal end portion of the linear member and the catheter shaft or the balloon, it is possible to reduce the outer diameter on the distal end side. Thus, the balloon catheter can further improve the crossability when the balloon is caused to move to the constricted location of the blood vessel.

In the aspect, the urging member may be disposed further toward the inflation region side of the balloon than the movement portion. In this case, the balloon catheter can suppress the outer diameter of a portion on the opposite side to the inflation region side of the balloon with respect to the movement portion of the linear member from being increased by the urging member. Therefore, the balloon catheter can improve the crossability when the balloon is caused to move to the constricted location of the blood vessel and when the balloon is pulled out from the blood vessel.

In the aspect, the urging member may be a coil spring wound around a part of the catheter shaft. In this case, the balloon catheter can effectively suppress the increase in the outer diameter due to the urging member (the coil spring). Further, in comparison to a case in which, for example, a cylindrical resin member is used as the urging member in place of the coil spring, it is possible to alleviate a decrease in the urging force due to deterioration over time.

In the aspect, an end portion of a wire of the coil spring may be bonded to a portion of the wire other than the end portion of the wire. In this case, the balloon catheter can suppress the end portion of the wire of the coil spring from becoming caught on the blood vessel.

In the aspect, the end portion of the wire of the coil spring may have a rounded shape. In this case, the balloon catheter can suppress the end portion of the wire of the coil spring from becoming caught on the blood vessel.

In the aspect, a covering member may be configured to cover the coil spring from outside. In this case, the covering member restricts the coil spring from coming into contact with the blood inside the blood vessel. Therefore, the balloon catheter can suppress a blood clot from adhering to the coil spring.

In the aspect, a part of the urging member may be bonded to the catheter shaft using an adhesive. In this case, it is possible to easily create the balloon catheter in which the part of the urging member is bonded to the catheter shaft.

### Brief description of Drawings

Fig. 1 is a side view of a balloon catheter 10.
Fig. 2 is a side view of a balloon 3 and a linear member 4 in a deflated state.
Fig. 3 is a cross-sectional view in the direction of arrows along a line A - A shown in Fig. 2.
Fig. 4 is a cross-sectional view of the balloon 3 and the linear member 4 in the deflated state.
Fig. 5 is a side view of the balloon 3 and the linear member 4 in an inflated state.
Fig. 6 is a cross-sectional view in the direction of arrows along a line B - B shown in Fig. 5.
Fig. 7 is a cross-sectional view of the balloon 3 and the linear member 4 in the inflated state.
Fig. 8 is an enlarged side view of the vicinity of a first mounting member 21A when the balloon 3 is in an enlarged state.
Fig. 9 is an enlarged side view of the vicinity of the first mounting member 21A when the balloon 3 is in the deflated state.
Fig. 10 is an enlarged view of an end portion 5B of a wire 5A of a first urging member 5.
Fig. 11 is an enlarged view of the end portion 5B of the wire 5A of the first urging member 5 according to a modified example.

### Description of Embodiments

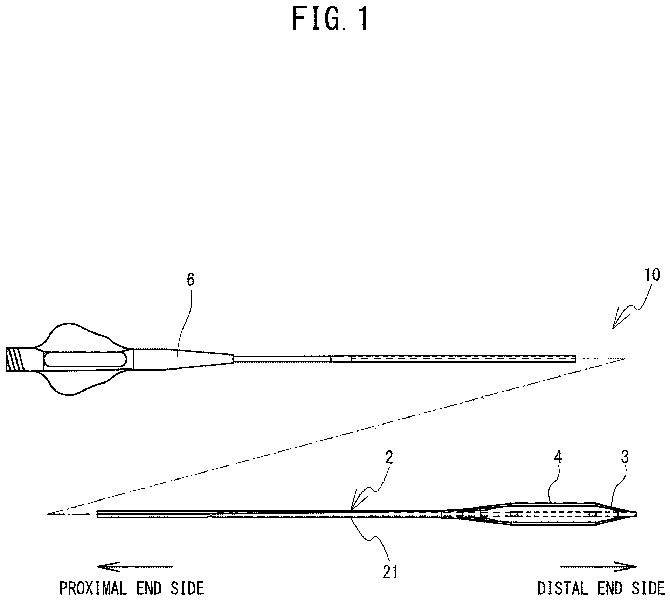

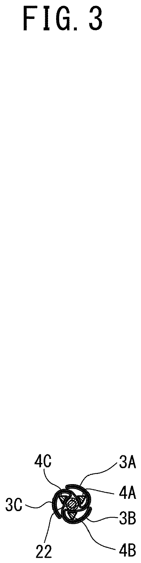

An embodiment of the present invention will be explained with reference to the drawings. As shown in Fig. 1, a balloon catheter 10 has a catheter shaft 2, a balloon 3, and linear members 4A, 4B, and 4 C (refer to Fig. 3, hereinafter collectively referred to as a "linear member 4"). The balloon 3 is connected to an end portion on one side of the catheter shaft 2. The linear member 4 is disposed on the outside of the balloon 3 in an inflated state. The balloon catheter 10 is used in a state in which a hub 6 is connected to an end portion on the other side of the catheter shaft 2. The hub 6 can supply compressed fluid to the balloon 3 via the catheter shaft 2. Hereinafter, the end (of both ends) of the catheter shaft 2 on the one side is referred to as a "distal end." The end (of both ends) of the catheter shaft 2 on the other side is referred to as a "proximal end." A direction extending along the catheter shaft 2 is referred to as an "extending direction." In a direction orthogonal to the extending direction, a side closer to the center of the cross section of the catheter shaft 2 is referred to as an "inner side," and a side away from the center of the cross section of the catheter shaft 2 is referred to as an "outer side."

### Catheter shaft 2

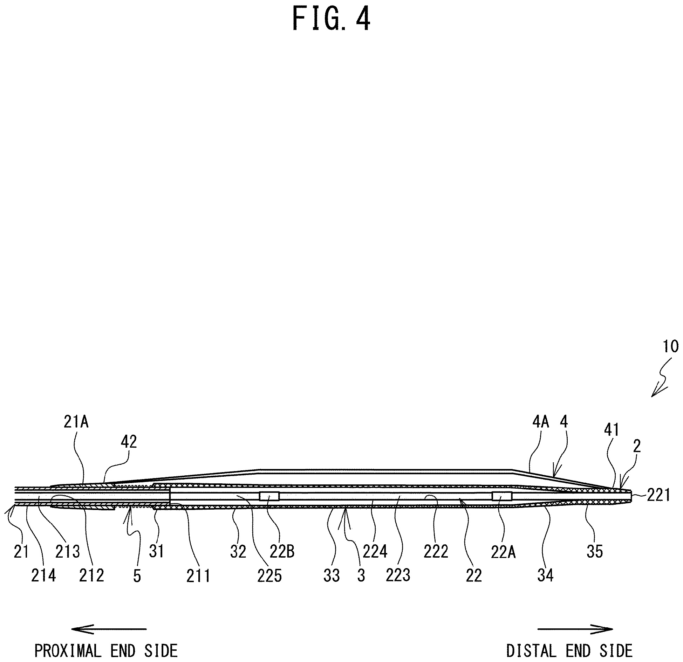

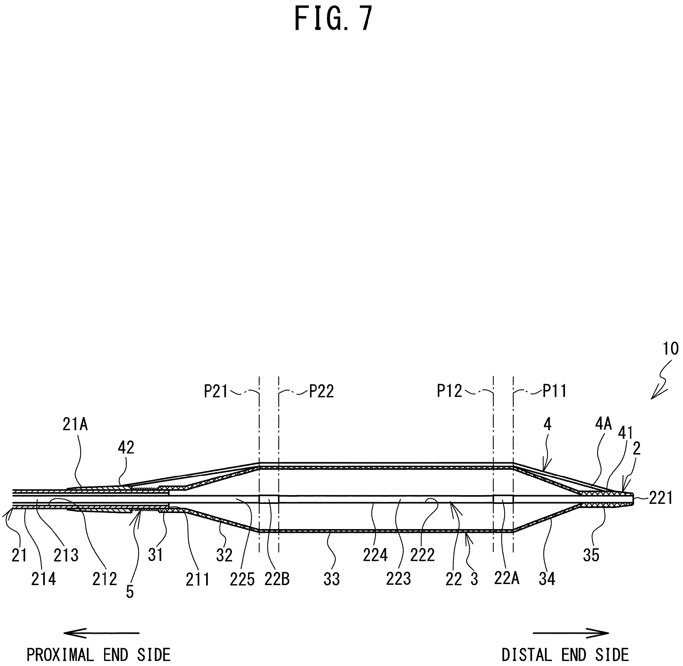

As shown in Fig. 4 and Fig. 7, the catheter shaft 2 has an outer tube 21 and an inner tube 22. The outer tube 21 and the inner tube 22 are both flexible tubular members. The outer tube 21 has a space (hereinafter referred to as a "lumen 213") surrounded by an inner surface 212, which is a surface on the inner side of the outer tube 21. The inner tube 22 has a space (hereinafter referred to as a "lumen 223") surrounded by a surface (hereinafter referred to as an "inner surface 222") on the inner side of the inner tube 22. The outer tube 21 and the inner tube 22 are formed of a polyamide resin. The inner diameter of the outer tube 21 is larger than the outer diameter of the inner tube 22.

Apart from a predetermined portion on the distal end side, the inner tube 22 is disposed inside the lumen 213 of the outer tube 21. The predetermined portion on the distal end side of the inner tube 22 protrudes toward the distal end side from an end (hereinafter referred to as a "distal end 211") on the distal end side of the outer tube 21. Therefore, an end (hereinafter referred to as a "distal end 221") on the distal end side of the inner tube 22 is disposed further toward the distal end side than the distal end 211 of the outer tube 21. Hereinafter, the predetermined portion on the distal end side of the inner tube 22 is referred to as a "protruding portion 225." Radiopaque markers (hereinafter simply referred to as "markers") 22A and 22B are fitted to the protruding portion 225 of the inner tube 22. The markers 22A and 22B are formed of an alloy of platinum and iridium. The markers 22A and 22B are fixed to an outer peripheral surface (hereinafter referred to as an "outer surface 224") of the inner tube 22, as a result of cylindrical members formed of the above-described material being crimped onto the protruding portion 225 of the inner tube 22. The markers 22A and 22B have a predetermined length in the extending direction. The markers 22A and 22B do not allow the passage of radiation. The marker 22A is disposed further toward the distal end side than the marker 22B. The markers 22A and 22B are separated from each other in the extending direction.

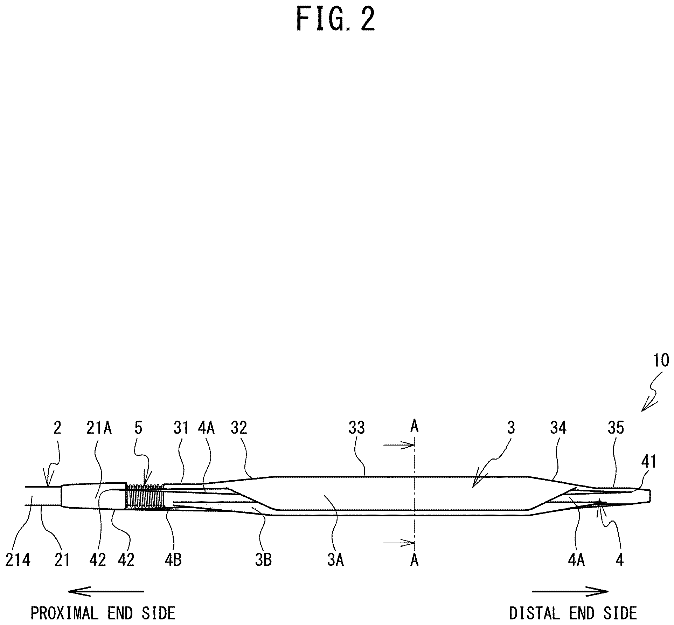

As shown in Fig. 2, Fig. 4, Fig. 5, and Fig. 7, a first mounting member 21A is mounted on a portion of an outer peripheral surface (hereinafter referred to as an "outer surface 214") of the outer tube 21, the portion being further toward the proximal end side than the distal end 211. The first mounting member 21A has a cylindrical shape. The first mounting member 21A can move along the extending direction. The inner diameter of the first mounting member 21A is larger than the outer diameter of the outer tube 21. A thermoplastic resin, such as a polyamide resin or the like, is used as the material of the first mounting member 21A.

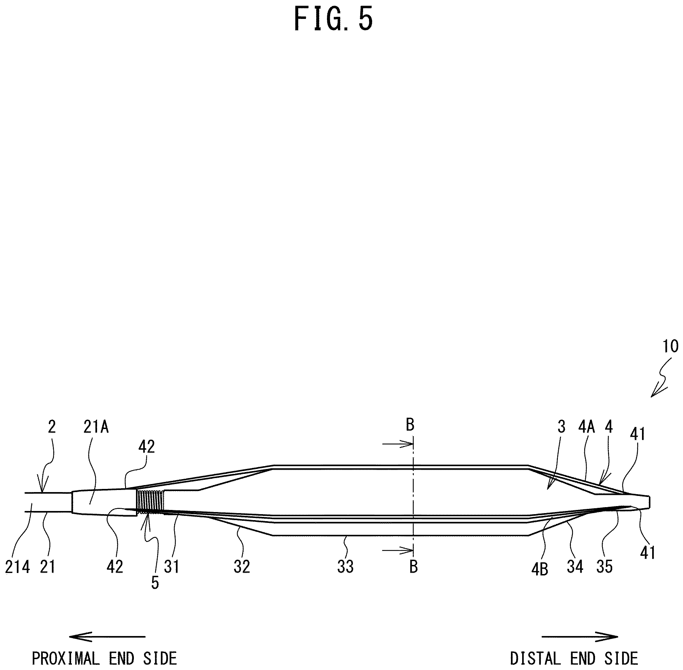

As shown in Fig. 4 and Fig. 7, the compressed fluid supplied from the hub 6 (refer to Fig. 1) flows through a space between the inner surface 212 of the outer tube 21 and the outer surface 224 of the inner tube 22. The balloon 3 inflates in accordance with the supply of the compressed fluid (refer to Fig. 5 to Fig. 7). A guide wire that is not shown in the drawings is inserted through the lumen 223 of the inner tube 22.

### Balloon 3

As shown in Fig. 2 to Fig. 4, the balloon 3 deflates to the inner side when the compressed fluid is not supplied. Meanwhile, as shown in Fig. 5 to Fig. 7, the balloon 3 inflates to the outer side when the compressed fluid is supplied. The balloon 3 is formed of a polyamide resin. As shown in Fig. 2, Fig. 4, Fig. 5, and Fig. 7, the balloon 3 includes a proximal end side leg portion 31, a proximal end side cone region 32, an inflation region 33, a distal end side cone region 34, and a distal end side leg portion 35. The proximal end side leg portion 31, the proximal end side cone region 32, the inflation region 33, the distal end side cone region 34, and the distal end side leg portion 35 respectively correspond to portions of the balloon 3 divided into five in the extending direction. The length of the inflation region 33 in the extending direction is longer than the respective lengths in the extending direction of the proximal end side leg portion 31, the proximal end side cone region 32, the distal end side cone region 34, and the distal end side leg portion 35.

As shown in Fig. 4 and Fig. 7, the proximal end side leg portion 31 is connected, by thermal welding, to the outer surface 214 of the outer tube 21, at a portion located further toward the proximal end side than the distal end 211 and further toward the distal end side than the portion on which the first mounting member 21A is mounted. The proximal end side cone region 32 is adjacent to the distal end side of the proximal end side leg portion 31. The inflation region 33 is adjacent to the distal end side of the proximal end side cone region 32. The distal end side cone region 34 is adjacent to the distal end side of the inflation region 33. The distal end side leg portion 35 is adjacent to the distal end side of the distal end side cone region 34. The distal end side leg portion 35 is connected, by thermal welding, to the outer surface 224 of the protruding portion 225 of the inner tube 22, at a portion located further toward the proximal end side than the distal end 221. The proximal end side leg portion 31, the proximal end side cone region 32, the inflation region 33, the distal end side cone region 34, and the distal end side leg portion 35 are disposed side by side in that order from the proximal end side toward the distal end side. The proximal end side cone region 32, the inflation region 33, the distal end side cone region 34, and the distal end side leg portion 35 cover the protruding portion 225 of the inner tube 22 from outside.

As shown in Fig. 2 to Fig. 4, the balloon 3 is a tri-fold balloon. In the deflated state, the balloon 3 forms three pleats. As shown in Fig. 3, in the deflated state, the balloon 3 is folded over so as to form three pleats 3A, 3B, and 3C. Each of the pleats 3A, 3B, and 3C is wrapped around the periphery of the protruding portion 225 of the inner tube 22. In this state, the pleat 3A covers the linear member 4A, which will be described later, from outside. The pleat 3B covers the linear member 4B, which will be described later, from outside. The pleat 3C covers the linear member 4C, which will be described later, from outside. The pleats 3A, 3B, and 3C are also called "flaps" and "wings."

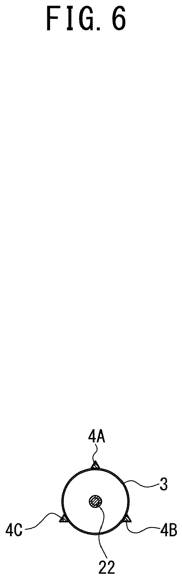

The balloon 3 in the inflated state will be explained with reference to Fig. 5 to Fig. 7. As shown in Fig. 6, the cross-sectional shape of the balloon 3 is circular. As shown in Fig. 5 and Fig. 7, the proximal end side cone region 32 has a tapered shape. The diameter of the proximal end side cone region 32 increases continuously and linearly from the proximal end side toward the distal end side. The diameter of the inflation region 33 is the same across the whole length in the extending direction. The distal end side cone region 34 has a tapered shape. The diameter of the distal end side cone region 34 decreases continuously and linearly from the proximal end side toward the distal end side. In other words, the diameter of the cross section of the balloon 3 changes in a stepped manner between the proximal end side cone region 32, the inflation region 33, and the distal end side cone region 34. The outer shape of the inflation region 33 becomes largest in the balloon 3.

As shown in Fig. 7, a position of a boundary of the inflation region 33 on the distal end side, in other words, a position of a boundary between the inflation region 33 and the distal end side cone region 34 is aligned, in the extending direction, with a position P11 of an end portion on the distal end side of the marker 22A. A position of a boundary of the inflation region 33 on the proximal end side, in other words, a position of a boundary between the inflation region 33 and the proximal end side cone region 32 is aligned, in the extending direction, with a position P21 of an end portion on the proximal end side of the marker 22B.

### Linear member 4

The linear member 4 will be explained with reference to Fig. 4 to Fig. 9. The linear member 4 is a monofilament-shaped elastic body. The linear member 4 has a restoring force with respect to bending deformation. The linear member 4 is formed of a polyamide resin. The linear member 4 has a triangular cross section. The cross section is a surface that is perpendicular to the extending direction of the linear member 4. The linear members 4A, 4B, and 4C have the same shape. The linear member 4 extends along the extending direction.

As shown in Fig. 4, Fig. 5, and Fig. 7, an end portion (hereinafter referred to as a "distal end portion 41") on the distal end side of the linear member 4 is connected, by thermal welding, further toward the distal end side than the inflation region 33 of the balloon 3, more specifically, to the outer peripheral surface of the distal end side leg portion 35 of the balloon 3. The distal end portions 41 of each of the linear members 4A, 4B, and 4C are connected, respectively, to positions that divide the outer peripheral surface of the distal end side leg portion 35 of the balloon 3 into three equal parts in the circumferential direction.

An end portion (hereinafter referred to as a "proximal end portion 42") on the proximal end side of the linear member 4 is connected, by thermal welding, further toward the proximal end side than the inflation region 33 of the balloon 3, more specifically, to the outer peripheral surface of the first mounting member 21A. The proximal end portions 42 of each of the linear members 4A, 4B, and 4C are connected, respectively, to positions that divide the outer peripheral surface of the first mounting member 21A into three equal parts in the circumferential direction. The proximal end portion 42 of the linear member 4 can move in the extending direction when the first mounting member 21A moves in the extending direction along the outer surface 214 of the outer tube 21. Note that the linear member 4 is not connected to the balloon 3 at any portion other than the distal end portion 41 and the proximal end portion 42. In the present embodiment, the proximal end portion 42 corresponds to a "movement portion" of the present invention.

The linear member 4 is disposed between the distal end position 41 and the proximal end position 42 so as to straddle the inflation region 33 of the balloon 3. As shown in Fig. 6, when the balloon 3 is in the inflated state, the linear members 4A, 4B, and 4C extend linearly in the extending direction, respectively, at positions that divide the outer peripheral surface of the inflation region 33 of the balloon 3 into three approximately equal parts in the circumferential direction.

### First urging member 5

As shown in Fig. 2, Fig. 4, Fig. 5, and Fig. 7, the balloon catheter 10 is provided with a first urging member 5. The first urging member 5 is disposed further toward the distal end side than the first mounting member 21A. In other words, the first urging member 5 is disposed on the inflation region 33 side of the balloon 3 with respect to the proximal end portion 42 of the linear member 4 that is connected to the first mounting member 21A. The first urging member 5 is a compression coil spring that is wound around the outer tube 21 of the catheter shaft 2. A coil spring made of SUS (stainless steel) is used as the first urging member 5. The inner diameter of the first urging member 5 is substantially the same as the outer diameter of the outer tube 21. A part on the distal end side of the first urging member 5, more specifically, a portion positioned on the most distal end side among respective portions obtained when the first urging member 5 is divided into three equal parts in the extending direction, is bonded to the outer surface 214 of the outer tube 21 using an adhesive. A known medical UV cure adhesive is used as the adhesive. In this case, the part on the distal end side of the first urging member 5 cannot move in the extending direction with respect to the catheter shaft 2. Meanwhile, a portion on the proximal end side of the first urging member 5, more specifically, a portion of the first urging member 5 that is not bonded by the adhesive can move in the extending direction with respect to the catheter shaft 2. Therefore, the first urging member 5 can be elastically deformed at the portion that is not bonded by the adhesive.

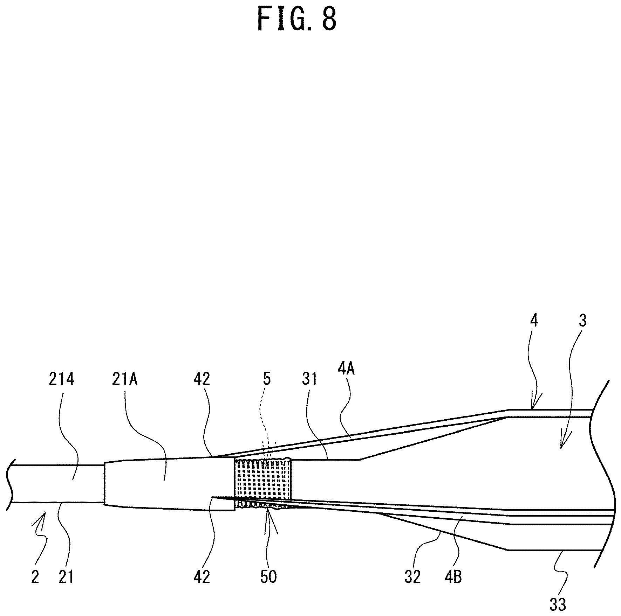

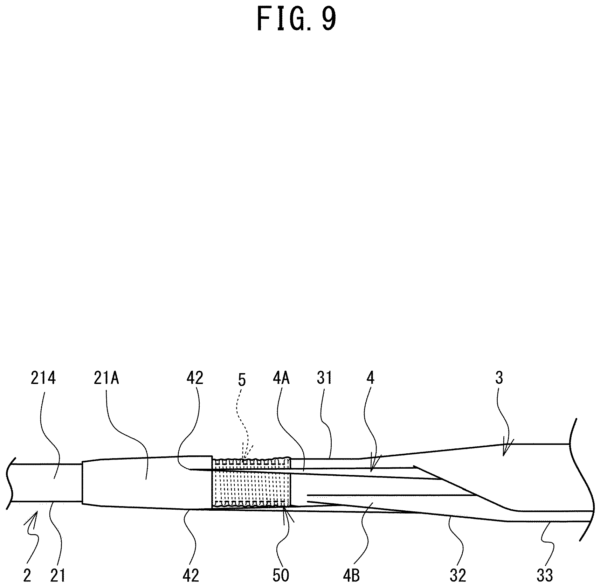

As shown in Fig. 8, and Fig. 9, an end portion on the proximal end side of the first urging member 5 can come into contact with and separate from an end portion on the distal end side of the first mounting member 21A. The first urging member 5 urges the first mounting member 21A in a direction toward the proximal end side. Therefore, the proximal end portion 42 of the linear member 4 connected to the first mounting member 21A is urged by the first urging member 5 in a direction away from the inflation region 33 of the balloon 3.

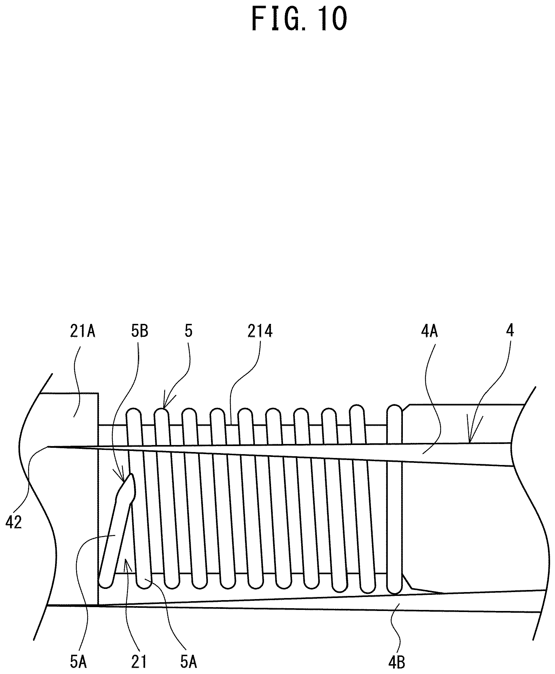

The first urging member 5 is covered by a covering member 50 from outside. The covering member 50 is a cylindrical member made of a resin. The covering member 50 is firmly attached to the first urging member 5 from outside. Therefore, the whole of the first urging member 5 is not exposed. As shown in Fig. 10, both the distal end side and the proximal end side of an end portion 5B of a wire 5A of the first urging member 5 are welded to a part of the adjacent wire 5A. Thus, a corner created by the end portion 5B of the wire 5A does not jut out sharply and has a rounded shape.

### Movement of linear member 4 in accordance with inflation/deflation of balloon 3

A state of the linear member 4 when the balloon 3 inflates as a result of the compressed fluid being supplied from the hub 6 will be explained. In accordance with the inflation of the balloon 3, the linear member 4 receives, from the balloon 3, a force in a direction toward the outside. The linear member 4 tries to move to the outside (refer to Fig. 7). The distal end portion 41 of the linear member 4 is connected to the distal end side leg portion 35 of the balloon 3. Therefore, a force in a direction approaching the inflation region 33 of the balloon 3 (a direction toward the distal end side) acts on the proximal end portion 42 of the linear member 4. Due to the force, as shown in Fig. 8, the first mounting member 21A connected to the proximal end portion 42 of the linear member 4 moves toward the distal end side along the extending direction. Therefore, a central portion of the linear member 4 moves to the outside, and the proximal end portion 42 of the linear member 4 moves toward the distal end side. The central portion of the linear member 4 is a portion of the linear member 4 between the proximal end portion 42 and the distal end portion 41. Note that the first urging member 5 is disposed on the distal end side of the first mounting member 21A. The first urging member 5 urges the first mounting member 21A in the direction away from the inflation region 33 of the balloon 3 (the direction toward the proximal end side). Specifically, when the balloon 3 is in a completely deflated state, the first urging member 5 urges the first mounting member 21A toward the proximal end side. Therefore, when the balloon 3 inflates, the first mounting member 21A moves toward the distal end side against the urging force in the direction toward the proximal end side applied by the first urging member 5.

A state of the linear member 4 when the balloon 3 deflates as a result of the compressed fluid being discharged from the balloon 3 in the inflated state will be explained. When the balloon 3 deflates, the force in the direction toward the outside that acts on the linear member 4 is weakened. At this time, as shown in Fig. 9, the first mounting member 21A connected to the proximal end portion 42 of the linear member 4 moves toward the proximal end side due to the urging force in the direction toward the proximal end side received from the first urging member 5. Thus, the linear member 4 approaches the protruding portion 225 of the inner tube 22. The warping of the linear member 4 is suppressed by the first mounting member 21A moving toward the proximal end side in response to the urging force of the first urging member 5. The linear member 4A is covered from the outside by the pleat 3A. The linear member 4B is covered from the outside by the pleat 3B. The linear member 4C is covered from the outside by the pleat 3C (refer to Fig. 3).

### Main operations and effects of embodiment

When the inflation region 33 of the balloon 3 inflates, the linear member 4 moves to the outside. Therefore, when the inflation region 33 inflates in a state in which the balloon 3 is disposed at a constricted location of a blood vessel, the balloon catheter 10 can cause the linear member 4 to act on the constricted location of the blood vessel. The proximal end portion 42 of the linear member 4 is connected to the first mounting member 21A that can move along the extending direction. The proximal end portion 42 of the linear member 4 moves to the balloon 3 side in accordance with the inflation of the balloon 3. Thus, the balloon catheter 10 can suppress the inflation of the balloon 3 from being obstructed by the linear member 4.

The first urging member 5 urges the first mounting member 21A in the direction away from the inflation region 33 of the balloon 3. Therefore, when the balloon 3 returns from the inflated state to the deflated state, the proximal end portion 42 of the linear member 4 connected to the first mounting member 21A moves in the direction away from the inflation region 33 of the balloon 3 along the extending direction, in response to the urging force received from the first urging member 5. In this case, the warping of the linear member 4 is suppressed when the balloon 3 is in the deflated state. Therefore, when the balloon 3 is in the deflated state, the balloon catheter 10 can suppress crossability (passing ability) from deteriorating due to the warping of the linear member 4. Since the first urging member 5 urges the first mounting member 21A toward the proximal end side, the proximal end portion 42, of the linear member 4, that is connected to the first mounting member 21A moves toward the proximal end side along the extending direction. The central portion of the linear member 4 moves so as to approach the catheter shaft 2. In accordance with this, the linear member 4 presses the balloon 3 toward the catheter shaft 2. More specifically, when the balloon 3 deflates, the linear member 4 assists the balloon 3 to deflate, in concert with the first urging member 5. Thus, the balloon catheter 10 can deflate the balloon 3 to be smaller in comparison to a configuration in which the first urging member 5 is not provided.

A part on the distal end side of the first urging member 5 is bonded to the outer surface 214 of the outer tube 21 of the catheter shaft 2. For example, when the first urging member 5 is held on the catheter shaft 2 by a stopper provided on the outer surface 214 of the outer tube 21 of the catheter shaft 2, the rigidity of the balloon catheter 10 becomes larger at a portion of the stopper than at the other portions. In the balloon catheter 10, the larger the change in the rigidity in the extending direction, the more easily torsion or twist (kinks) occurs. In contrast to this, in the balloon catheter 10, the first urging member 5 is directly bonded to the catheter shaft 2. Thus, the balloon catheter 10 can reduce the extent of the change in the rigidity in the extending direction. Therefore, the balloon catheter 10 can suppress the occurrence of kinks due to the change in the rigidity in the extending direction. Since the part of the first urging member 5 is bonded to the outer surface 214 of the outer tube 21, even when the first urging member 5 becomes caught on a stent or the like disposed inside the blood vessel, the first urging member 5 is inhibited from coming apart from the catheter shaft 2 inside the blood vessel.

The first mounting member 21A, to which the proximal end portion 42 of the linear member 4 is connected, is provided on the proximal end side with respect to the inflation region 33 of the balloon 3. In this case, the balloon catheter 10 can suppress the outer diameter on the distal end side from being increased by the first mounting member 21A. Therefore, the balloon catheter 10 can improve the crossability when the balloon 3 is caused to move toward the constricted location of the blood vessel.

The distal end portion 41 of the linear member 4 is connected to the outer peripheral surface of the distal end side leg portion 35 of the balloon 3. In other words, the distal end portion 41 of the linear member 4 is connected to the catheter shaft 2 via the distal end side leg portion 35 of the balloon 3. In contrast to this, there is a case in which an elastically deformable member is interposed between the distal end portion 41 of the linear member 4 and the catheter shaft 2 so that the linear member 4 can easily move when the balloon 3 inflates or deflates. In this case, the outer diameter on the distal end side of the balloon catheter 10 increases, and there is a possibility of deterioration in the crossability. In contrast to this, in the balloon catheter 10, the distal end portion 41 of the linear member 4 is directly connected to the catheter shaft 2 via the balloon 3. Therefore, it is possible to reduce the outer diameter on the distal end side of the balloon catheter 10. Thus, the balloon catheter 10 can further improve the crossability when the balloon 3 is caused to move to the constricted location of the blood vessel.

The first urging member 5 is provided further toward the distal end side than the first mounting member 21A, in other words, on the inflation region 33 side of the balloon 3 with respect to the proximal end portion 42 of the linear member 4 that is connected to the fist mounting member 21A. In this case, the balloon catheter 10 can suppress the outer diameter of a portion located further toward the proximal end side than the first mounting member 21A, in other words, the outer diameter of a portion on the opposite side to the inflation region 33 side of the balloon 3 with respect to the proximal end portion 42 of the linear member 4, from being increased by the first urging member 5. Therefore, the balloon catheter 10 can improve the crossability when the balloon is caused to move to the constricted location of the blood vessel and when the balloon is pulled out from the blood vessel.

The first urging member 5 is the compression coil spring that is wound around a part of the outer tube 21 of the catheter shaft 2. In this case, the balloon catheter 10 can suppress, to the minimum, the increase in the outer diameter as a result of the first urging member 5 being provided. Further, in comparison to a case in which, for example, a cylindrical resin member is used as the first urging member 5 in place of the coil spring, it is possible to alleviate the decrease in the urging force due to deterioration over time.

The end portion 5B of the wire 5A of the first urging member 5 is welded to a part of the adjacent wire 5A. Thus, the corner created by the end portion 5B of the wire 5A does not jut out sharply and has a rounded shape. In this case, the balloon catheter 10 can suppress the end portion 5B of the wire 5A from becoming caught on the blood vessel.

The first urging member 5 is covered by the covering member 50 from the outside. In this case, the covering member 50 restricts the first urging member 5 from coming into contact with the blood inside the blood vessel. Therefore, the balloon catheter 10 can suppress a blood clot from adhering to the first urging member 5.

A part on the distal end side of the first urging member 5 is bonded to the outer surface 214 of the outer tube 21 using the adhesive. In this case, it is possible to easily create the balloon catheter 10 in which the part of the first urging member 5 is bonded to the catheter shaft 2.

### Modified examples

The present invention is not limited to the above-described embodiment and various modifications are possible. In the above-described embodiment, the position of the boundary on the distal end side of the inflation region 33 is aligned, in the extending direction, with the position P11 of the end portion on the distal end side of the marker 22A. The position of the boundary on the proximal end side of the inflation region 33 is aligned, in the extending direction, with the position P21 of the end portion on the proximal end side of the marker 22B. The position of the boundary on the distal end side of the inflation region 33 need not necessarily be aligned completely, in the extending direction, with the position P11 on the distal end side of the marker 22A. For example, the position of the boundary on the distal end side of the inflation region 33 may be aligned, in the extending direction, with any position between the position P11 of the end portion on the distal end side of the marker 22A and a position P12 of the end portion on the proximal end side thereof. The position of the boundary on the proximal end side of the inflation region 33 need not necessarily be aligned completely, in the extending direction, with the position P21 on the proximal end side of the marker 22B. For example, the position of the boundary on the proximal end side of the inflation region 33 may be aligned, in the extending direction, with any position between the position P21 of the end portion on the proximal end side of the marker 22B and a position P22 of the end portion on the distal end side thereof. It is sufficient that the positions of the marker 22A and the marker 22B respectively correspond to the position of the boundary on the distal end side of the inflation region 33 and the position of the boundary on the proximal end side of the inflation region 33. The number of the markers is not limited to two and may be one, or three or more. When the number of the markers is one, the marker is provided at a position corresponding to the center position of the inflation region 33. Further, the markers 22A and 22B may be made of resin into which a radiopaque material is mixed.

In the above-described embodiment, each of the boundary portion between the proximal end side cone region 32 and the inflation region 33 of the balloon 3 in the inflated state and the boundary portion between the inflation region 33 and the distal end side cone region 34 may be curved. In this case, for example, with respect to the positions of each of the boundaries, when a plurality of virtual planes that tangent the respectively curved boundary portions are defined, positions of the boundary portions that touch the virtual plane, of the plurality of virtual planes, that forms an acute angle of 45 degrees with the extending direction may be the positions of each of the boundaries. In the above-described embodiment, each of the proximal end side cone region 32 and the distal end side cone region 34 is a region whose diameter changes linearly from the proximal end side toward the distal end side. However, each of the proximal end side cone region 32 and the distal end side cone region 34 may be a region whose diameter changes in a curved manner from the proximal end side toward the distal end side. One of the proximal end side cone region 32 and the distal end side cone region 34 may be the region whose diameter changes in the curved manner and the other may be the region whose diameter changes linearly. The thickness of the balloon 3 may change along the extending direction. For example, the thickness of each of the proximal end side cone region 32 and the distal end side cone region 34 may gradually increase toward the inflation region 33. A degree of increase in the thickness may be different between the proximal end side cone region 32 and the distal end side cone region 34. The thickness of one of the proximal end side cone region 32 and the distal end side cone region 34, only, may change. The diameter of the inflation region 33 may change along the extending direction. For example, the diameter of the inflation region 33 may increase or decrease toward the central portion in the extending direction. The thickness of the inflation region 33 may increase or decrease toward the central portion in the extending direction. The balloon 3 may be formed using polyester or polyethylene.

In the above-described embodiment, the linear member 4 is a member that extends substantially linearly along the extending direction. The shape of the linear member 4 is not limited to the above-described embodiment. For example, the linear member 4 may be a member that extends in a spiral shape along the extending direction. The linear member 4 may have a cross section that is not triangular. For example, the linear member 4 may have a circular cross section. The number of the linear members 4 may be a number other than three. The linear member 4 need not necessarily be an elastic body. The linear member 4 may be a metal member.

In the above-described embodiment, the first urging member 5 of the balloon catheter 10 may be a tension coil spring. The tension coil spring may be disposed further toward the proximal end side than the first mounting member 21A, in other words, further toward the proximal end side than the proximal end portion 42 of the linear member 4 that is connected to the first mounting member 21A. In the above-described embodiment, the balloon catheter 10 may be further provided with a second mounting member at the distal end portion of the protruding portion 225 of the inner tube 22. The second mounting member may be movable in the extending direction along the outer surface 224 of the inner tube 22. The distal end portion 41 of the linear member 4 may be connected to the second mounting member. In this way, the distal end portion 41 of the linear member 4 may be configured to be movable in the extending direction. A second urging member, which urges the second mounting member toward the opposite side from the inflation region 33 side of the balloon 3, may be provided. The second urging member may be a compression coil spring that is provided on the inflation region 33 side of the balloon 3 with respect to the distal end portion 41 of the linear member 4 that is connected to the second mounting member, or may be a tension coil spring that is provided on the opposite side to the inflation region 33 of the balloon 3 with respect to the distal end portion 41 of the linear member 4 that is connected to the second mounting member. The balloon catheter 10 may only have the second mounting member and the second urging member, and need not necessarily have the first mounting member 21A and the first urging member 5. In this case, the distal end portion 41 corresponds to the "movement portion" of the present invention. When the distal end portion 41 and the proximal end portion 42 are respectively configured to be movable, the distal end portion 41 and the proximal end portion 42 correspond to the "movement portion" of the present invention. The first urging member 5 may be a coil spring coated by DLC (diamond-like carbon). The first urging member 5 and the second urging member are not limited to the coil springs, and may be other elastic members that can apply the urging force to the mounting member. For example, the first urging member 5 and the second urging member may be made of an elastic resin (such as rubber).

The first urging member 5 may be configured to not urge the first mounting member 21A toward the proximal end side when the balloon 3 is in the completely deflated state. For example, the first urging member 5 may be disposed so as to be in contact with, but not urge the first mounting member 21A when the balloon 3 is in the completely deflated state. Further, the first urging member 5 may be disposed so as not to be in contact with the first mounting member 21A when the balloon 3 is in the completely deflated state. In this case, the first urging member 5 may be configured to urge the first mounting member 21A toward the proximal end side when the first mounting member 21A moves by a predetermined distance toward the distal end side in accordance with the inflation of the balloon 3. Further, the proximal end portion of the first urging member 5 and the distal end portion of the first mounting member 21A may be bonded together.

The distal end portion 41 of the linear member 4 may be connected to a portion, of the inner tube 22 of the catheter shaft 2, that is further toward the distal end side than the portion to which the distal end side leg portion 35 of the balloon 3 is connected. In other words, the distal end portion 41 of the linear member 4 may be directly connected to the catheter shaft 2. The catheter shaft 2 may be provided with a distal end tip that is bonded to the distal end of the inner tube 22. For example, the distal end tip is a member that is more flexible than the inner tube 22. The distal end tip extends from the distal end 221 of the inner tube 22 toward the distal end side. A through hole, which is communicated with the lumen 223 of the inner tube 22, is formed in the distal end tip. The outer diameter of the distal end tip may be substantially the same as the outer diameter of the inner tube 22, or may become smaller toward the distal end. A joint portion at which the inner tube 22 and the distal end tip are joined may be positioned further toward the proximal end side than the distal end side leg portion 35 of the balloon 3, or may be positioned further toward the distal end side. When the joint portion of the inner tube 22 and the distal end tip is positioned further toward the distal end side than the distal end side leg portion 35, the distal end side leg portion 35 may be bonded to the outer surface of the joint portion of the inner tube 22 and the distal end tip. The distal end portion 41 of the linear member 4 may be bonded to the outer surface of the distal end tip.

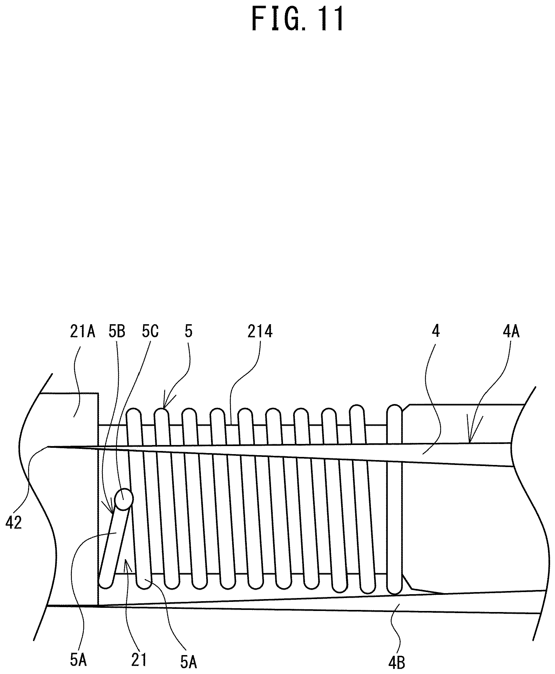

The end portion 5B of the wire 5A of the first urging member 5 need not necessarily be welded to a part of the adjacent wire 5A. For example, as shown in Fig. 11, a spherical body portion 5C having a hemispherical shape may be connected to the end portion 5B of the wire 5A of the first urging member 5. Since the spherical body portion 5C can cause the end portion 5B of the wire 5A to have a rounded shape, it is possible to suppress the end portion 5B of the wire 5A from becoming caught on the blood vessel. Note that the shape of the end portion 5B of the wire 5A of the first urging member 5 is not limited to the rounded shape and may be angular.

The covering member 50 may cover the whole of the first urging member 5 or may cover a part of the first urging member 5. The covering member 50 may be a cylindrical member in which a spiral slit is formed. As a result of the slit being formed, changes in the rigidity of the covering member 50 in the extending direction can be alleviated. The balloon catheter 10 need not necessarily be provided with the covering member 50.

A part on the distal end side of the first urging member 5 may be bonded to the catheter shaft 2 without using the adhesive. For example, a part of the distal end portion of the first urging member 5 may be bonded by being crimped onto the outer tube 21 of the catheter shaft 2. The portion at which the first urging member 5 is bonded to the outer tube 21 need not necessarily be the portion positioned on the most distal end side among the respective portions of the first urging member 5 divided into three equal parts. It is sufficient that the first urging member 5 be provided with an urging portion between the bonded portion with the outer tube 21 and the first mounting member 21A, the urging portion urging the first mounting member 21A toward the proximal end side. For example, the central portion of the first urging member 5 in the extending direction may be bonded to the outer tube 21. The first urging member 5 may be bonded to the outer tube 21 at a plurality of portions.

In the above-described embodiment, the example is given of the catheter shaft 2 that has the outer tube 21 and the inner tube 22. In the present invention, the catheter shaft 2 need not necessarily have the outer tube 21 and the inner tube 22. For example, the catheter shaft 2 may have only one flexible tube.

## Claims

1. A balloon catheter comprising:
a catheter shaft extending from a proximal end to a distal end;
a balloon connected to the catheter shaft and including an inflation region that can be inflated;
a linear member including a distal end portion positioned further toward the distal end side than the inflation region, and a proximal end portion positioned further toward the proximal end side than the inflation region, at least one of the distal end portion and the proximal end portion being movable along an extending direction of the catheter shaft; and
an urging member configured to be capable of urging a movement portion, which is one of the distal end portion and the proximal end portion and which is movable in the extending direction, in a direction away from the inflation region of the balloon, the urging member being partially bonded to the catheter shaft.

2. The balloon catheter according to claim 1, wherein
the movement portion is the proximal end portion.

3. The balloon catheter according to claim 2, wherein
the linear member is bonded to the catheter shaft or the balloon at the distal end portion.

4. The balloon catheter according to any one of claims 1 to 3, wherein
the urging member is disposed further toward the inflation region side of the balloon than the movement portion.

5. The balloon catheter according to any one of claims 1 to 4, wherein
the urging member is a coil spring wound around a part of the catheter shaft.

6. The balloon catheter according to claim 5, wherein
an end portion of a wire of the coil spring is bonded to a portion of the wire other than the end portion of the wire.

7. The balloon catheter according to claim 5 or 6, wherein
the end portion of the wire of the coil spring has a rounded shape.

8. The balloon catheter according to any one of claims 5 to 7, further comprising:
a covering member configured to cover the coil spring from outside.

9. The balloon catheter according to any one of claims 1 to 8, wherein
a part of the urging member is bonded to the catheter shaft using an adhesive.
